(19) Europäisches Patentamt
European Patent Office
Office européen des brevets

(11) Veröffentlichungsnummer : **0 504 113 A1**

(12) # EUROPÄISCHE PATENTANMELDUNG

(21) Anmeldenummer : **92810174.0**

(22) Anmeldetag : **06.03.92**

(51) Int. Cl.[5] : **C07D 495/06**, C07D 517/06, H01B 1/12, // (C07D495/06, 339:00, 339:00), (C07D517/06, 345:00, 345:00)

(30) Priorität : **15.03.91 CH 798/91**

(43) Veröffentlichungstag der Anmeldung :
**16.09.92 Patentblatt 92/38**

(84) Benannte Vertragsstaaten :
**CH DE FR GB LI NL**

(71) Anmelder : **CIBA-GEIGY AG**
**Klybeckstrasse 141**
**CH-4002 Basel (CH)**

(72) Erfinder : **Hauenstein, Kurt**
**Giffig 130**
**CH-5305 Unterendingen (CH)**
Erfinder : **Mayer, Carl W.**
**Steingrubenweg 224**
**CH-4125 Riehen (CH)**

(54) **Verfahren zur Herstellung von peridichalkogenierten Aromaten.**

(57) Verfahren zur Herstellung von peridichalkogenierten Aromaten, bei dem in einer ersten Stufe in einem amidischen Lösungsmittel ein Alkalimetallsulfid, -selenid oder -tellurid hergestellt wird, das man in einer zweiten Verfahrensstufe mit einem wenigstens zwei benachbarte Halogenatome enthaltenden Perihalogenaromaten umsetzt, dadurch gekennzeichnet, dass man in der ersten Verfahrensstufe bei erhöhter Temperatur eine Alkalimetallbase mit elementarem Schwefel, Selen oder Tellur umsetzt.

EP 0 504 113 A1

Die Erfindung betrifft ein Verfahren zur Herstellung von peridichalkogenierten Aromaten, bei dem man in einer ersten Verfahrensstufe ein Alkalimetallchalkogenid durch Umsetzung von Schwefel, Selen oder Tellur mit einer Alkalimetallbase in Gegenwart eines Säureamids als Lösungsmittel erzeugt und dieses in einer zweiten Verfahrensstufe mit einem Perihalogenaromaten reagieren lässt.

Perichalkogenierte Aromaten sind Elektronendonatoren, die mit Elektronenakzeptoren kristalline Radikalionensalze mit hoher elektrischer Leitfähigkeit bilden. Zur Herstellung von perichalkogenierten Aromaten wurde von K.A. Balodis et al. im Zhurnal Organicheskoi Khimii, Vol. 15, No. 2, Seiten 391 bis 393 [(1979), englische Übersetzung, Plenum Publishing Corporation, 1979] vorgeschlagen, zum Beispiel 5,6, 11,12-Tetrachlortetracen mit Natriumdiselenid bei erhöhter Temperatur umzusetzen, das durch die direkte Reaktion von Natriummetall mit Selen in Dimethylformamid als Lösungsmittel erhalten wird. Das 5,6,11,12-Tetraselenotetracen wird hierbei in einer Ausbeute von 50 % erhalten. D. J. Sandman et al. beschreiben die gleiche Methode zur Herstellung von 5,6, 11,12-Tetratellu(Ausbeute 13 %) in Organometallics, Vol. 1, Seiten 739 bis 742 (1982). B. K. Kruptsov et al. beschreiben in CA 77(13): 87350a, dass Lithium bei 80 °C mit Dimethylformamid reagiert.

Diese beschriebenen Methoden haben verschiedene Nachteile, die eine Durchführung im industriellen Masstab verhindern. Durch die Reaktion von Alkalimetallen mit amidischen Lösungsmitteln können keine eindeutigen stöchiometrischen Reaktionsverhältnisse eingestellt werden, so dass bei grösseren Ansätzen Ausbeuten und Produktqualität nicht reproduziert werden können. Die Reaktionslösungen sind durch die Bildung von Nebenprodukten sehr viskos und die Produkte werden in Form von sehr feinkristallinen Niederschlägen gebildet, so dass deren Abtrennung durch Filtration äusserst schwierig und langwierig ist. Die Handhabung grösserer Mengen von Alkalimetallen ist zudem problematisch und erfordert entsprechende Sicherheitsmassnahmen.

Es wurde nun gefunden, dass man chalkogenierte Aromaten in kürzeren Reaktionszeiten mit wesentlich höheren Ausbeuten und in hohen Reinheitsgraden unter Vermeidung der Verwendung von Alkalimetallen und bei relativ niedrigeren Temperaturen erhalten kann, wenn man die Alkalimetallchalkogenide durch Umsetzung von Alkalimetallbasen mit elementarem Schwefel, Selen oder Tellur in amidischen Lösungsmitteln herstellt. Dies ist sehr überraschend, da es völlig unbekannt war, dass Alkalimetallbasen in solchen Lösungsmitteln elementare Chalkogene praktisch stöchiometrisch zu entsprechenden Chalkogeniden zu reduzieren vermögen, und ebenso nicht bekannt ist, wie diese Reaktion abläuft.

Gegenstand der Erfindung ist ein Verfahren zur Herstellung von peridichalkogenierten Aromaten, bei dem in einer ersten Stufe in einem amidischen Lösungsmittel ein Alkalimetallsulfid, -selenid oder -tellurid hergestellt wird, das man in einer zweiten Verfahrensstufe mit einem wenigstens zwei benachbarte Halogenatome enthaltenden Perihalogenaromaten umsetzt, das dadurch gekennzeichnet ist, dass man in der ersten Verfahrensstufe bei erhöhter Temperatur eine Alkalimetallbase mit elementarem Schwefel, Selen oder Tellur umsetzt.

Chalkogen bedeutet hierbei die Elemente Schwefel, Selen und Tellur.

Die Reaktionstemperatur kann in der ersten Verfahrensstufe zum Beispiel von 30 °C bis zur Rückflusstemperatur des verwendeten Lösungsmittels, bevorzugt 30 °C bis 200 °C und besonders bevorzugt 50 °C bis 150 °C betragen.

Bevorzugte Alkalimetallbasen sind solche von Lithium, Natrium und Kalium, wobei Natrium besonders bevorzugt ist. Geeignete Basen sind zum Beispiel die Hydroxide, Carbonate, Hydrogencarbonate und Alkoholate von primären, sekundären und tertiären Alkanolen mit bevorzugt 1 bis 18, besonders bevorzugt 1 bis 12, und insbesondere bevorzugt 1 bis 6 C-Atomen. In einer bevorzugten Ausführungsform werden die Alkalimetallalkoholate von $C_1$-$C_{18}$-Alkanolen verwendet, besonders bevorzugt die entsprechenden Natriumalkoholate. Die Natriumalkoholate leiten sich insbesondere von $C_1$-$C_6$-Alkanolen ab. Ganz besonders bevorzugt ist Natriummethylat. In einer besonders bevorzugten Ausführungsform sind die Alkalimetallbasen aus der Gruppe NaOH, $NaHCO_3$, $Na_2CO_3$, $NaOCH_3$ und $KOC(CH_3)_3$ ausgewählt.

Bei den amidischen Lösungsmitteln handelt es sich bevorzugt um N-dialkylierte Carbonsäureamide, wobei die Carbonsäuren bevorzugt 1 bis 6, besonders bevorzugt 1 bis 3 C-Atome enthalten, um N-alkylierte Lactame und um N-peralkylierte Amide von anorganischen Sauerstoffsäuren, zum Beispiel Phosphorsäure oder Schwefelsäure. Die Alkylgruppen am N-Atom enthalten bevorzugt 1 bis 4 C-Atome, besonders bevorzugt 1 oder 2 C-Atome. Einige Beispiele sind N,N-Dimethylformamid, N,N-Diethylformamid, N,N-Di-n-propylformamid, N,N-Dimethylacetamid, N-Methylpyrrolidon, Tetramethylharnstoff und Hexamethylphosphorsäuretriamid. Bevorzugte Lösungsmittel sind Tetramethylhamstoff, N-Methylpyrrolidon und N,N-Dimethylacetamid. Besonders bevorzugt wird N,N-Dimethylacetamid verwendet.

Bei der Umsetzung der elementaren Chalkogene Schwefel, Selen und Tellur mit den Alkalimetallbasen werden Verbindungen der Formel $M_2X_2$ gebildet, worin M ein Alkalimetall und X Schwefel, Selen oder Tellur bedeuten. Die Alkalimetallbasen und Chalkogene werden bevorzugt in stöchiometrischen Verhältnissen eingesetzt.

Die Umsetzung wird zweckmässig unter Inertgasatmosphäre und unter Feuchtigkeitsausschluss durchgeführt, was auch für die zweite Verfahrensstufe gilt. Vorteilhaft ist eine Stickstoff- oder Argonatmosphäre.

Im allgemeinen kann bei der ersten Verfahrenstufe so vorgegangen werden, dass man die Alkalimetallbase und das Chalkogen im Lösungsmittel vorlegt und anschliessend erwärmt, bis die Bildung der Dialkalimetalldichalkogenide beendet ist. Die Reaktionszeiten liegen etwa im Bereich von einer halben bis höchstens fünf, im allgemeinen höchstens drei Stunden. Die zweite Verfahrensstufe wird dann direkt anschliessend durchgeführt.

Bei den in der zweiten Verfahrensstufe verwendeten perihalogenierten Aromaten handelt es sich bevorzugt um peribromierte und besonders bevorzugt um perichlorierte Aromaten, wobei mindestens zwei benachbarte Peristellungen halogeniert sind. Peristellungen liegen nur in kondensierten aromatischen Verbindungen vor. Es kann sich um heteroaromatische und bevorzugt kohlenwasserstoffaromatische Verbindungen handeln, die mindestens 10 C-Atome und bis zu 30 C-Atome, bevorzugt 10 bis 18 C-Atome enthalten. Beispiele für Aromaten sind Naphthalin, Anthracen und Tetracen.

In einer bevorzugten Ausführungsform entsprechen die perihalogenierten Aromaten der Formel I,

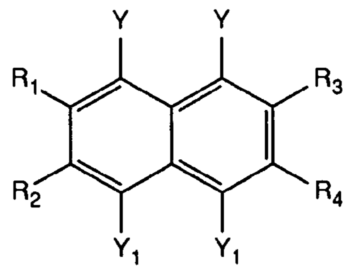

(I),

aus denen man perichalkogenierte Verbindungen der Formel II herstellt,

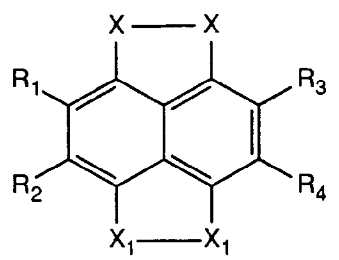

(II),

worin Y für Halogen, besonders für Cl, $Y_1$ für H oder Halogen, besonders Cl, X für S, Se oder Te und $X_1$ für H, S, Se oder Te stehen, und $R_1$, $R_2$, $R_3$ und $R_4$ unabhängig voneinander H, $C_1$-$C_4$-Alkyl, $C_1$-$C_4$-Alkoxy, $C_1$-$C_4$-Alkylthio, Halogen, besonders F, Cl und Br, CN, $CF_3$, $(CH_3)_3Si$, Di-($C_1$-$C_6$-Alkyl)N, CO($C_1$-$C_6$-Alkoxy) oder OCO($C_1$-$C_6$-Alkoxy) bedeuten, oder $R_1$ und $R_2$ oder $R_3$ und $R_4$ je zusammen den Rest -CH=C($R_7$)-C($R_8$)=CH- darstellen, oder $R_1$ und $R_2$ zusammen diesen Rest darstellen und $R_3$ und $R_4$ zusammen den Rest -CH=C($R_7$)-C($R_8$)=CH- bedeuten, wobei $R_8$, $R_6$, $R_7$ und $R_8$ unabhängig die Bedeutung von $R_1$ bis $R_4$ haben.

$Y_1$ steht bevorzugt für Halogen, besonders Cl, und $X_1$ steht bevorzugt für S, Se oder Te.

Besonders bevorzugte perihalogenierte Aromaten entsprechen den Formeln Ia und Ib,

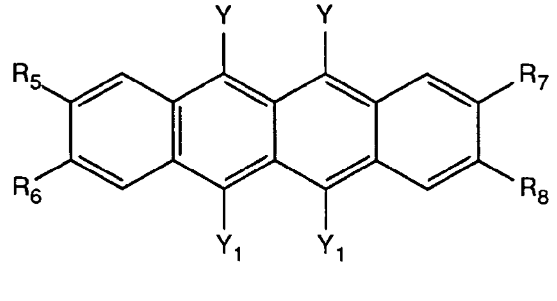

(Ia),

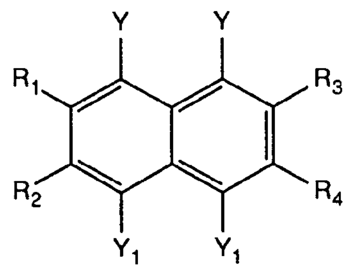

(Ib),

worin Y für Cl und $Y_1$ für H oder Cl stehen, und $R_1$ bis $R_4$ sowie $R_5$ bis $R_8$ unabhängig voneinander H, F, Cl, $CF_3$, $CH_3$, $CH_3O$, $C_2H_5O$, $CH_3S$, $C_2H_5S$, $(CH_3)_3Si$, $C(O)OCH_3$ oder $C(O)OC_2H_5$ darstellen. Besonders bevorzugt bedeuten $R_1$ bis $R_4$ sowie $R_5$ bis $R_8$ H.

Die Verbindungen der Formel I, a und Ib sind bekannt oder können nach bekannten Verfahren hergestellt werden.

Die zweite Verfahrensstufe des erfindungsgemässen Verfahrens wird zweckmässig direkt nach Beendigung der ersten Verfahrensstufe ohne Isolierung der Alkalimetallchalkogenide im gleichen Reaktionsmedium durchgeführt. Hierbei kann das Reaktionsmedium zuvor abgekühlt und nach Zugabe der perihalogenierten Aromaten wieder erwärmt werden. Man kann die perihalogenierten Aromaten aber auch zur heissen Reaktionsmischung zugeben. Die perihalogenierten Aromaten können vorteilhaft in einem gegebenenfalls heissen Lösungsmittel gelöst oder in Substanz zum Reaktonsgemisch gegeben werden. Zu diesem Zeitpunkt kann das Reaktionsgemisch auch durch Zugabe von Lösungsmittel verdünnt werden. Zweckmässig wird das gleiche Lösungsmittel verwendet wie in der ersten Verfahrensstufe.

In einer Variante des erfindungsgemässen Verfahrens kann man auch so vorgehen, dass man die Alkalimetallbase und das elementare Chalkogenid zusammen mit dem Perihalogenaromaten in dem Lösungsmittel vorlegt, das Gemisch erwärmt und ausreagieren lässt.

Die Reaktionstemperatur kann zum Beispiel von 30 °C bis zur Siedetemperatur des Lösungsmittels betragen, bevorzugt 30 bis 200 °C, und besonders bevorzugt 50 bis 150 °C. Die Reaktionszeiten betragen im allgemeinen 15 Minuten bis zu etwa 2 Stunden.

Die Reaktanden werden vorteilhaft in solchen Mengenverhältnissen eingesetzt, dass pro 2 benachbarte Perihalogenatome in einem Mol Aromaten mindestens 1 Mol Alkalimetallchalkogenid ($M_2X_2$) vorliegt. Es hat sich als günstig erwiesen, einen Überschuss an Alkalimetallchalkogenid zu verwenden, zum Beispiel bis 5 Mol, bevorzugt bis zu 3 Mol Alkalimetallchalkogenid pro 2 benachbarte Perihalogenatome in einem Mol Aromat.

Die Isolierung der gebildeten Peridichalkogenaromaten erfolgt in an sich bekannter Weise durch Filtration, wobei vorteilhaft ist, dass grobkristalline Produkte gebildet werden, die leicht abgetrennt werden können. Die Reinigung der Produkte kann in üblicher Weise durch Auswaschen mit Lösungsmitteln und gegebenenfalls Umkristallisation und/oder Sublimation erfolgen.

Die erfindungsgemäss hergestellten Peridichalkogenaromaten bilden mit Elektronenakzeptoren ionische Radikalsalze, die eine hohe elektrische Leitfähigkeit aufweisen (organische Metalle) und als elektrische Leiter Anwendung finden, zum Beispiel als Elektroden.

Die nachfolgenden Beispiele erläutern die Erfindung näher.

Beispiel 1: Herstellung von 5,6,11,12-Tetrathiotetracen.

In einem 200 ml Sulfierkolben werden unter Argon 1,83 g (5 mmol) 5,6, 11,12-Tetrachlortetracen, 1,76 g (21 mmol) Natriumhydrogencarbonat und 0,8 g Schwefel in 75 ml N,N-Dimethylacetamid eine Stunde am Rückfluss erhitzt. Danach lässt man abkühlen und filtriert den gebildeten schwarzen Niederschlag ab. Der Niederschlag wird mit N,N-Dimethylacetamid, Aceton, Wasser und nochmals Aceton gewaschen und über Nacht im Hochvakuum bei 60 °C getrocknet. Die Ausbeute beträgt 1,237 g (70,3 %) und der Gehalt an der Titelverbindung 84 %. Der Gehalt wird spektroskopisch in einer Lösung von 1,2,4-Trichlorbenzol mit der Absorptionsbande bei 705 nm bestimmt.

Beispiel 2: Herstellung von 5,6,11,12-Tetrathiotetracen

Es wird wie in Beispiel 1 verfahren, aber als Base 1,13 g (21 mmol) Natriummethylat verwendet und die Reaktionszeit auf 30 Minuten verkürzt. Die Ausbeute beträgt 77,8 % und der spektroskopisch bestimmte Gehalt an der Titelverbindung beträgt 94,5 %.

Beispiel 3: Herstellung von 5,6,11,12-Tetraselenotetracen.

In einem 350 ml Sulfierkolben wird unter Argon ein Gemisch aus 1,896 g (24 mmol) Selen, 1,336 g (24 mmol) Natriummethylat und 50 ml N,N-Dimethylacetamid innerhalb von 30 Minuten bis zum Rückfluss erhitzt und eine Stunde bei dieser Temperatur belassen. Dann wird zu der gebildeten rotbraunen Suspension eine heisse Lösung von 1,83 g (5 mmol) 5,6,11,12-Tetrachlortetracen in 100 ml N,N-Dimethylacetamid auf einmal zugegeben. Die Reaktionsmischung verfärbt sich sofort grün. Man erhitzt weitere 20 Minuten am Rückfluss und kühlt dann auf 50 °C ab. Der kristalline schwarze Niederschlag wird abfiltriert, mit 50 ml N,N-Dimethylacetamid, und dann mit je 400 ml Wasser und Aceton gewaschen. Danach trocknet man den Rückstand über Nacht im Hochvakuum bei 60 °C. Man erhält 2,36 g (87,4 %) Rohprodukt mit einem spektroskopisch (Absorptionsbande bei 712 nm) ermittelten Gehalt an der Titelverbindung von mehr als 97 %.

Beispiel 4: Herstellung von 5,6,11,12-Tetraselenotetracen.

0,948 g (12 mMol) Selen und 0,668 g (12 mMol) Natriummethylat werden unter Argonatmosphäre und Rühren in 25 ml N-Methylpyrrolidon von Raumtemperatur auf 140 °C erwärmt und eine Stunde weitergerührt. Zu der entstandenen rotbraunen Suspension wird eine 140 °C heisse Lösung von 0,915 g (2,5 mMol) 5,6,11,12-Tetrachlortetracen in 50 ml N-Methylpyrrolidon auf einmal zugegeben. Nach 20 Minuten wird auf Raumtemperatur abgekühlt, der Niederschlag abgesaugt, mit 25 ml kaltem N-Methylpyrrolidon, 2 mal 100 ml Wasser und 2 mal 50 ml Aceton gewaschen und dann im Hochvakuum bei 40 °C getrocknet. Die Ausbeute beträgt 0,959 g (71 %) Rohprodukt mit einem Gehalt an der Titelverbindung von 93 %.

Beispiel 5: Herstellung von 5,6,11,12-Tetraselenotetracen.

Es wird wie in Beispiel 4 verfahren, aber unter Verwendung von Tetramethylharnstoff als Lösungsmittel. Man erhält 1,35 g Rohprodukt mit einem Gehalt an der Titelverbindung von 79 %.

Beispiel 6: Herstellung von 5,6,11,12-Tetraselenotetracen.

1,738 g (22 mMol) Selen und 2,655 g (23 mMol) Kalium-t-butylat werden in 50 ml Dimethylacetamid unter Argonatmosphäre während 30 Minuten auf 145 °C erwärmt. Zu der rotbraunen Suspension wird auf einmal eine heisse Lösung von 1,83 g (5 mMol) 5,6,11,12-Tetrachlortetracen in 100 ml Dimethylacetamid gegeben und dann bei 155 °C 20 Minuten gerührt. Man laässt auf 50 °C abkühlen, nutscht den schwarzen Niederschlag ab, und wäscht den Niederschlag mit 50 ml Dimethylacetamid, 400 ml Wasser und zweimal mit je 100 ml Aceton. Nach dem Trocknen im Hochvakuum bei 50 °C 1,59 g (58,9 %) Rohprodukt mit einem Gehalt an der Titelverbindung von 97 %.

Beispiel 7: Herstellung von 5,6,11,12-Tetraselenotetracen.

0,948 g (12 mMol) Selen, 1,008 g (12 mMol) $NaHCO_3$ und 1 ml Wasser werden in Dimethylacetamid 2 Stunden bei 130 °C unter Argonatmosphäre gerührt. Danach wird eine 130 °C heisse Lösung von 0,915 g (2,5 mMol) 5,6,11,12-Tetrachlortetracen in 50 ml Dimethylacetamid zugegeben und 3 Stunden gerührt weitergerührt. Dann lässt man Abkühlen, nutscht den Niederschlag ab, wäscht mit 25 ml Dimethylacetamid, 100 ml Wasser und 100 ml Aceton und trocknet dann im Hochvakuum bei 50 °C. Man erhält 0,887 g (65,7 %) Rohprodukt mit einem Gehalt an der Titelverbindung von 69 %.

Beispiel 8: Herstellung von 5,6,11,12-Tetraselenotetracen.

0,948 g (12 mMol) Selen und 0,636 g (8 mMol) Natriumcarbonat werden unter Argonatmosphäre in 25 ml Dimethylacetamid und 3 ml Wasser auf 140 °C erwärmt und 1 Stunde bei dieser Temperatur gerührt. Dann wird eine 130 °C heisse Lösung von 0,915 g (2,5 mMol) 5,6,11,12-Tetrachlortetracen in 50 ml Dimethylacetamid auf einmal zugegeben und 4 Stunden bei 140 °C weitergerührt. Nach dem Abkühlen auf Raumtemperatur wird der schwarze Niederschlag abfiltriert, mit 25 ml Dimethylacetamid, 100 ml Wasser und 100 ml Aceton gewaschen und dann im Hochvakuum bei 50 °C getrocknet. Man erhält 0,84 g (62 %) Rohprodukt mit einem Gehalt an der Titelverbindung von 58 %.

Beispiel 9: Herstellung von 5,6,11,12-Tetraselenotetracen.

480 mg (12 mMol) NaOH werden in 1 ml Wasser gelöst und zu einer Suspension von 0,948 g (12 mMol) Selen in 25 ml Dimethylacetamid gegeben. Dann rührt man das Gemisch 1 Stunde bei 140 °C. Danach wird eine 130 °C heisse Lösung von 0,915 g (2,5 mMol) 5,6,11,12-Tetrachlortetracen in 50 ml Dimethylacetamid auf einmal zugegeben und darauf 4 Stunden bei 150 °C unter Argonatmosphäre weitergerührt. Nach dem Abkühlen auf 50 °C wird der schwarze Niederschlag abfiltriert, mit 30 ml Dimethylacetamid, 200 ml Wasser und 200 ml Aceton gewaschen und dann im Hochvakuum bei 50 °C getrocknet. Man erhält 1,137 g (84 %) Rohprodukt mit einem Gehalt an der Titelverbindung von 88 %.

Beispiel 10: Herstellung von 2-Fluor-5,6,11,12-Tetraselenotetracen.

0,948 g (12 mMol) Selen und 0,668 g (12 mMol) Natriummethylat in 25 ml Dimethylacetamid werden unter Argonatmosphäre während 30 Minuten auf 150 °C erhitzt und eine Stunde bei dieser Temperatur gerührt. Anschliessend gibt man eine 150 °C heisse Lösung von 2,5 mMol 2-Fluor-5,6,11,12-Tetraselenotetracen in 50 ml Dimethylacetamid auf einmal zu. Man rührt noch 20 Minuten bei 150 °C und lässt dann auf Raumtemperatur abkühlen. Der schwarze Niederschlag wird abfiltriert, mit 20 ml Dimethylacetamid, 200 ml Wasser und 200 ml Aceton gewaschen und dann im Hochvakuum bei 50 °C getrocknet. Man erhält 1,142 g (81 %) Rohprodukt mit einem Gehalt an der Titelverbindung von 86 %.

Beispiel 11: Herstellung von 1,4,5,8-Tetraselenonaphthalin

0,948 g (12 mMol) Selen und 0,668 g (12 mMol) Natriummethylat in 25 ml Dimethylacetamid werden unter Argonatmosphäre während 30 Minuten auf 150 °C erhitzt und eine Stunde bei dieser Temperatur gerührt. Anschliessend gibt man eine 140 °C heisse Lösung von 2,5 mMol 1,4,5,8-Tetrachlornaphthalin in 50 ml Dimethylacetamid auf einmal zu. Man rührt noch 22 Stunden bei 150 °C und lässt dann auf Raumtemperatur abkühlen. Der braunschwarze Niederschlag wird abfiltriert, mit 20 ml Dimethylacetamid, 200 ml Wasser und 200 ml Aceton gewaschen und dann im Hochvakuum bei 50 °C getrocknet: Man erhält 0,953 g (86,6 %) Rohprodukt mit einem Gehalt an der Titelverbindung von praktisch 100 %.

Beispiel 12: Herstellung von 5,6,11,12-Tetratellurotetracen

Man verfährt wie in Beispiel 3, aber unter Verwendung von Tellur anstelle von Selen. Die Ausbeute an Rohprodukt und der Gehalt an der Titelverbindung entspricht im wesentlichen jenen gemäss Beispiel 3.

## Patentansprüche

1. Verfahren zur Herstellung von peridichalkogenierten Aromaten, bei dem in einer ersten Stufe in einem amidischen Lösungsmittel ein Alkalimetallsulfid, -selenid oder -tellurid hergestellt wird, das man in einer zweiten Verfahrensstufe mit einem wenigstens zwei benachbarte Halogenatome enthaltenden Perihalogenaromaten umsetzt, dadurch gekennzeichnet, dass man in der ersten Verfahrensstufe bei erhöhter Temperatur eine Alkalimetallbase mit elementarem Schwefel, Selen oder Tellur umsetzt.

2. Verfahren gemäss Anspruch 1, dadurch gekennzeichnet, dass die Reaktionstemperatur in der ersten Verfahrensstufe von 30 °C bis zur Rückflusstemperatur des verwendeten Lösungsmittels reicht.

3. Verfahren gemäss Anspruch 2, dadurch gekennzeichnet, dass die Reaktionstemperatur 30 bis 200 °C beträgt.

4. Verfahren gemäss Anspruch 1, dadurch gekennzeichnet, dass als Alkalimetallbasen solche von Lithium, Natrium oder Kalium verwendet werden.

5. Verfahren gemäss Anspruch 1, dadurch gekennzeichnet, dass als Alkalimetallbasen Hydroxide, Carbonate, Hydrogencarbonate und Alkoholate von primären, sekundären und tertiären Alkanolen verwendet werden.

6. Verfahren gemäss Anspruch 5, dadurch gekennzeichnet, dass die Alkanole 1 bis 18 C-Atome enthalten.

7. Verfahren gemäss Anspruch 5, dadurch gekennzeichnet, dass man Natriumalkoholate verwendet.

8. Verfahren gemäss Anspruch 7, dadurch gekennzeichnet, dass man Natriumalkoholate von $C_1$-$C_6$-Alkanolen verwendet.

9. Verfahren gemäss Anspruch 1, dadurch gekennzeichnet, dass als Alkalimetallbase Natriummethylat verwendet wird.

10. Verfahren gemäss Anspruch 1, dadurch gekennzeichnet, dass es sich bei dem amidischen Lösungsmittel um N-dialkylierte Carbonsäureamide, N-alkylierte Lactame und N-peralkylierte Amide von anorganischen Sauerstoffsäuren handelt.

11. Verfahren gemäss Anspruch 10, dadurch gekennzeichnet, dass es sich bei dem amidischen Lösungsmittel um N,N-Dimethylacetamid handelt.

12. Verfahren gemäss Anspruch 1, dadurch gekennzeichnet, dass man die Alkalimetallbasen und elementaren Chalkogene Schwefel, Selen oder Tellur in stöchiometrischen Mengenverhältnissen zur Bildung von Verbindungen der Formel $M_2X_2$ einsetzt, worin M ein Alkalimetall und X Schwefel, Selen oder Tellur bedeuten.

13. Verfahren gemäss Anspruch 1, dadurch gekennzeichnet, dass es sich bei den perihalogenierten Aromaten um peribromierte oder perichlorierte Aromaten handelt.

14. Verfahren gemäss Anspruch 1, dadurch gekennzeichnet, dass die perihalogenierten Aromaten heteroaromatische oder kohlenwasserstoffaromatische Verbindungen sind, die mindestens 10 C-Atome und bis zu 30 C-Atome enthalten.

15. Verfahren gemäss Anspruch 1, dadurch gekennzeichnet, dass die perihalogenierten Aromaten der Formel I entsprechen,

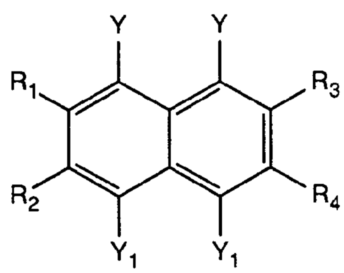

(I),

aus denen man perichalkogenierte Verbindungen der Formel II herstellt,

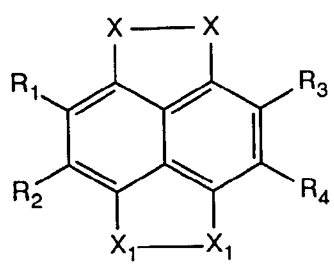

(II),

worin Y für Halogen, $Y_1$ für H oder Halogen, X für S, Se oder Te und $X_1$ für H, S, Se oder Te stehen, und $R_1$, $R_2$, $R_3$ und $R_4$ unabhängig voneinander H, $C_1$-$C_4$-Alkyl, $C_1$-$C_4$-Alkoxy, $C_1$-$C_4$-Alkylthio, Halogen, CN, $CF_3$, $(CH_3)_3Si$, Di-($C_1$-$C_6$-Alkyl)N, CO($C_1$-$C_6$-Alkoxy) oder OCO($C_1$-$C_6$-Alkoxy) bedeuten, oder $R_1$ und $R_2$ oder $R_3$ und $R_4$ je zusammen den Rest -CH=C($R_5$)-C($R_6$)=CH- darstellen, oder $R_1$ und $R_2$ zusammen diesen Rest darstellen und $R_3$ und $R_4$ zusammen den Rest -CH=C($R_7$)-C($R_5$)=CH- bedeuten, wobei $R_5$, $R_6$,

$R_7$ und $R_8$ unabhängig die Bedeutung von $R_1$ bis $R_4$ haben.

**16.** Verfahren gemäss Anspruch 15, dadurch gekennzeichnet, dass die perihalogenierten Aromaten den Formel Ia oder Ib entsprechen,

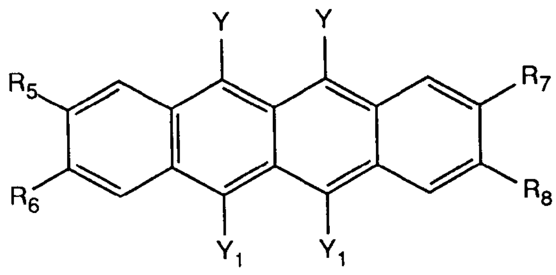

(Ia),

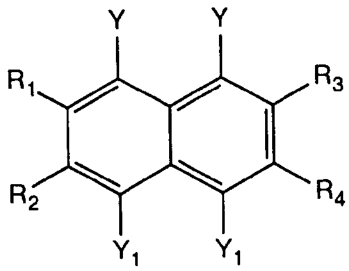

(Ib),

worin Y für Cl und $Y_1$ für H oder Cl stehen, und $R_1$ bis $R_4$ sowie $R_5$ bis $R_8$ unabhängig voneinander H, F, Cl, $CF_3$, $CH_3$, $CH_3O$, $C_2H_5O$, $CH_3S$, $C_2H_5S$, $(CH_3)_3Si$, $C(O)OCH_3$ oder $C(O)OC_2H_5$ darstellen.

**17.** Verfahren gemäss Anspruch 16, dadurch gekennzeichnet, dass $R_1$ bis $R_4$ und $R_5$ bis $R_8$ H bedeuten.

**18.** Verfahren gemäss Anspruch 1, dadurch gekennzeichnet, dass man die Alkalimetallbase und das elementare Chalkogenid zusammen mit dem Perihalogenaromaten in dem Lösungsmittel vorlegt, das Gemisch erwärmt und ausreagieren lässt.

**19.** Verfahren gemäss Anspruch 1, dadurch gekennzeichnet, dass die Reaktionstemperatur in der zweiten Verfahrensstufe von 30 °C bis zur Siedetemperatur des Lösungsmittels beträgt.

**20.** Verfahren gemäss Anspruch 1, dadurch gekennzeichnet, dass die Reaktanden in solchen Mengenverhältnissen eingesetzt werden, dass pro 2 benachbarte Perihalogenatome in einem Mol Aromaten mindestens 1 Mol Alkalimetallchalkogenid $M_2X_2$ vorliegt.

Europäisches Patentamt

# EUROPÄISCHER RECHERCHENBERICHT

Nummer der Anmeldung

EP 92 81 0174

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| Y | CHEMISTRY LETTERS.<br>Nr. 2, 1987, TOKYO JP<br>Seiten 315 - 316;<br>T. OTSUBO ET AL.: '1,4,5,8-Tetrachalcogeno-2,3,6,7-tetramethylnaphthalenes as new electron donors'<br>* Seite 315 * | 1 | C07D495/06<br>C07D517/06<br>H01B1/12<br>//(C07D495/06, 339:00,339:00)<br>(C07D517/06, 345:00,345:00) |
| A | | 1,10,11, 13-15, 19,20 | |
| Y | CHEMICAL ABSTRACTS, vol. 92, no. 4, 28. Januar 1980, Columbus, Ohio, US;<br>abstract no. 25135K,<br>K. L. PALANISWAMY ET AL.: 'Sodium sulfides and sodium sulfite'<br>Seite 171 ;<br>& IN-A-144029<br>* Zusammenfassung * | 1 | |
| A | | 1-5,12 | |
| A | SYNTHESIS.<br>Nr. 5, Mai 1984, STUTTGART DE<br>Seiten 439 - 442;<br>L. SYPER ET AL.: 'The convenient synthesis of organoselenium compounds'<br>* Seite 441 * | 1-5, 10-13, 18-20 | RECHERCHIERTE SACHGEBIETE (Int. Cl.5 )<br>C07D<br>H01B |

Der vorliegende Recherchenbericht wurde für alle Patentansprüche erstellt

| Recherchenort | Abschlußdatum der Recherche | Prüfer |
|---|---|---|
| DEN HAAG | 13 JULI 1992 | VOYIAZOGLOU D. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)